Europäisches Patentamt

European Patent Office

Office européen des brevets

(11) Publication number: **0 462 766 A1**

# EUROPEAN PATENT APPLICATION

(21) Application number: **91305428.4**

(22) Date of filing: **14.06.91**

(51) Int. Cl.⁵: **A61K 37/54,** // **(A61K37/54, 37:34)**

(30) Priority: **19.06.90 AR 317159**

(43) Date of publication of application: **27.12.91 Bulletin 91/52**

(84) Designated Contracting States: **DE FR GB IT SE**

(71) Applicant: **Altman, Raul 2008 Viamonte St. 1056 Buenos Aires (AR)**
Applicant: **Rouvier, Jorge Felix 2008 Viamonte St. 1056 Buenos Aires (AR)**

(72) Inventor: **Altman, Raul 2008 Viamonte St. 1056 Buenos Aires (AR)**
Inventor: **Rouvier, Jorge Felix 2008 Viamonte St. 1056 Buenos Aires (AR)**

(74) Representative: **Pendlebury, Anthony et al PAGE, WHITE & FARRER 54 Doughty Street London WC1N 2LS (GB)**

(54) **Composition useful in thrombolytic therapy.**

(57)    The present invention relates to thrombolytic therapy, and in particular to a composition for use in such therapy, said composition comprising a combination of (A) desmopressin (also known as DDAVP) and (B) a substance selected from streptokinase, urokinase, prourokinase, acylenzymes and acylated thrombolytic agents, and the use of such compositions.

EP 0 462 766 A1

Field of the Invention:

The clinical syndrome of acute myocardial infarction is caused by limitation of coronary blood flow of a magnitude and duration so as to result in necrosis of a significant quantity of myocardium. The coronary thrombosis is often responsible for the initiation of infarction and the thrombolytic agents restore angiographic patency to coronary vessels.

The present invention relates with a method useful in thrombolytic therapy, and with a composition applicable in such method. Furthermore, the invention relates with a kit containing the substances to be used in the above method.

In the early 1980's intracoronary streptokinase became popular as a technique to restore flow to the occluded coronary artery but unfortunately most of the trials have failed to show significant improvement in mortality because of delay involved in catheterizing patients. The streptokinase is used for obtaining thrombolytic effect at doses of 1,500,000-3,000,000 IU. With the intravenous route of administering thrombolytic agent therapy may be initiated early and widescale application. Plasminogen Activator Inhibitor (PAI) is a substance which blockades the tissue plasminogen activator (t-PA) and moderates its activity. At present streptokinase (at the above doses) and TPA are the substances mainly used in therapy to obtain a thrombolytic effect.

Background of the Invention:

Desmopressin (1-deamino-8-arginine vasopressin, DDAVP) is a synthetic analogue of the 1-arginine vasopressin. It can raise plasma level of factor VIII coagulant activity, von Willebrand factor and enhances fibrinolysis by increasing the plasma level of tissue plasminogen activator. Desmopressin increases the level of tissue plasminogen activator by causing them to be released from vascular endothelial cells. Desmopressin release plaminogen activator inhibitor 1 at the same time but the final balance is in the enhancement of the clot lysis direction. The concurrent release of urokinase-type plasminogen activator might eventually cooperate in the enhancement of fibrinolytic activity.

Desmopressin has few troublesome side effects. Consequently, the present invention relates with a composition resulting from the combination of desmopressin and streptokinase as a new thrombolytic regimen for acute myocardial infarction. The treatment trend to obtain a synergistic effect between the activation of fibrinolysis by streptokinase and the endogenous tissue plasminogen activator released from endothelium. DDAVP can be also combined in the extent of the compositions of this invention, with urokinase or pro-urokinase, or with acylenzymes or acylated thrombolytic agents such as the acylated derivatives of the streptokinase-plasminogen complex, BRL 16921 known as APSAC (acylplasminogen streptokinase complex). The therapy properties of these alternative substances are disclosed, for instance, in the following citations:

Urokinase: Kambara, H., Kawai, C., et al: Coronary thrombolysis with urokinase infusion in acute myocardial infarction:

Multicenter study in Japan, Cather Cardiovasc diagh 11:349-360, 1985.

Pro-urokinase: Van der Werf, F., Bobwhara M., Collen D.,; Coronary thrombolysis with human single chain urokinase-type plasminogen activator (pro-urokinase) in patients with acute myocardial infarction, Ann Int Med 104,304-309, 1986.

Acylenzymes: Green J., Harris, G.S. et al: Acyl-enzymes: a novel class of thrombolytic agents.In Thrombolysis biological and therapeutic properties of new thrombolytic agents. Church Livingstone, 1985.

ARSAC: Monnier, P., Sigwart, U. et al: Systemic thrombolysis within the first three hours of acute coronary thrombosis: APSAC versus streptokinase: a randomized study. Thromb Haemost 58(1) 1-676, Abstract 196, July 6th., 1987.

The substances of this invention can be added as a composition, or alternatively, one after each other. The ranges are of about 100,000 - 1,500,000 IU Streptokinase in combination with 4-40 µg DDAVP or 1-30 mg acylenzymes or 1-80 mg pro-urokinase or 100,000 - 3,000,000 IU urokinase.

The substances for carrying out the thrombolytic therapy of the invention can be included in a kit useful for providing the ingredients and eventually further elements useful for enabling the application of the cited ingredients.

The thrombolytic therapy trends to induce maximal local thrombolysis with minimal systemic effect in the blood.

To determining the incidence of the substances combination in the coagulation system and fibrinolytic system, assays were carried out to study their modifications by investigating specific parameters before and after administration of streptokinase and DDAVP at 30, 60, 120 and 240 minutes.

The studies in humans consisted of 22 patients, 6 women and 16 men admitted to the Coronary Care Unit having had chest pain characteristic of typical myocardial ischemia for 30 minutes, ST-segment elevation of

2

at least 2 mm in two or more consecutive leads, presentation within 12 hours in Group A and within 6 hours in Group A and within 6 hours in Group B after the onset of pain, age under 75 years and informed consent.
Patients characteristics are shown in Table 1

### Table 1

|  | GROUP A | GROUP B |
|---|---|---|
| Male | 7 | 9 |
| Female | 4 | 2 |
| Age (Mean) | 59.7 | 56.3 |
| Delay to starting therapy(min) | 259 | 205 |
| Risk Factors |  |  |
|    Hypertension | 7 | 8 |
|    Smoking | 5 | 9 |
|    Diabetes | 1 | 1 |
|    Hypercholesterolemia | 2 | 2 |
| Infarct location |  |  |
|    Anterior | 4 | 6 |
|    Inferior and/or posterior | 5 | 1 |
|    Multiple location· | 2 | 4 |
| Killip Class: |  |  |
|    I | 8 | 9 |
|    II | 2 | 2 |
|    III | – | – |
|    IV | 1 | – |
| Previous AMI | 2 | 1 |
| Mean reperfusion time (min.) | 177+/-26 | 158+/-15 |

After basal blood sampling for coagulation and fibrinolysis studies, patients were treated with intravenous nitroglycerin during 15 minutes at the maximal dose not associated with a significant increase in heart rate or associated with a more than 10% decrease in systolic pressure. If the ST-segment elevation was not resolved, all patients received aspirin plus beta blockers (atenolol 5mg) intravenously in absence of absolute contraindications. Thrombolytic treatment started as soon as the diagnostic criteria indicated an acute myocardial infarction. Patients enrolled in Group A were treated with 150,000 IU of streptokinase in bolus followed by 24µg of desmopressin in 3-5 minutes. Beta blockers and aspirin were given during the in-hospital follow-up. No heparin was used after thrombolytic therapy. The group B was treated with 250,000 IU of streptokinase during 5 minutes infusion followed by desmopressin in 3 minutes intravenously. Seta blockers and aspirin were also given in this group. Heparin was administered intravenously to maintain the activated partial thromboplastin time between 2-2.5, the control value starting after the last blood sample was taken (240 minutes from the beginning of thrombolytic therapy). The heparin was stopped after a therapeutic level of oral anti-coagulant was reached (International Normalized Ratio 2-3). It was also stopped 2 hours before the angiographic study and restarted after it. Oral anticoagulant was indicated at the same time.

Fibrinogen level was measured according to the functional assay of Clauss (Gerinnungsphysiologische Schnellmethode zur Bestimmung des Fibrinogens. Acta Haematol 1957; 17:237-46). Fibrinogen/fibrin degradation products were assayed using the staphyloccocal clumping test (Sigma, St. Louis, Mo. USA).

The Clinical results are shown in Table 2.

Table 2

|  | n | % |
|---|---|---|
| REPERFUSION | 17/22 | 77.3 |
| PATENCY | 12/15 | 80 |
| REINFARCTION | 1/22 | 4.5 |
| POST-INFARCT ANGINA | 4/22 | 18.2 |
| BLEEDING COMPLICATIONS | 0/22 |  |
| IN-HOSPITAL MORTALITY | 1/22 | 4.5 |
| MORTALITY AFTER HOSPITAL DISCHARGE | 0/21 |  |

The results showed the following (with reference to Table 3a):
1) The fibrinolytic activity significantly increased (even before 30 minutes) and the effect still maintained even after four hours.
Fibrinogen/fibrin degradation products (FDP) were substantially increased in relation with the shortening of the euglobulin lysis time (Table 1).
2) Fibrinogen (FIB) declined to levels not dangerous for the hemostasis of the patient.
3) Euglobulin lysis time (ELT) decreased at the first 30 minutes and then gradually increased.
4) The peak increase of FDP was related with the maximal concentration of D-Dimer (D-D).
With reference to Table 4:
5) it is evident that the level of tissue plasminogen activator (t-PA) concentration observed during therapy raised from 14 +/- 6ng/ml to 29.7 +/7.7 ng/ml at 30 minutes with an increase of 109%. All patients excepting one responded adequately to DDAVP.
6) PAI-1 concentration remained unchanged. Tissue plasminogen activator/tissue plasminogen activator inhibitor 1 complex was above normal values before starting the therapy (6.5 +/- 2.6ng/ml; normal 1.3 +/0.2ng/ml). An increase of 112% was detected at 30 minutes (p 0.001) which remains between 77-91% up to the last blood sample. The free tissue plasminogen activator in plasma (functional active) increased from 7.8ng/ml at basal to 16.6ng/ml at 240 minutes (Table 4b).
7) Although urokinase type plasminogen activator could also be released by desmopressin from endothelial cells, it hard contributed to the increased fibrinolytic activity because its plasmatic activity decreased during the study (Table 4a).
8) von Willebrand factor is also released from endothelium by desmopressin: a moderate but significant increase of 32% of the protein and 49% as ristocetin cofactor in comparison to the basal values were assayed, respectively (Group A, Table 6).

Table 6

| MIN | V (%) | VIII:c(%) | vWag(%) | R Co(%) |
|---|---|---|---|---|
| BASAL | 84+/-18 | 83+/-10 | 94+/-19 | 97+/-20 |
| 30 | 52+/-19 | 73+/-15 | 132+/-22 | 136+/-33 |
| 60 | 58+/-17 | 59+/-19 | 129+/-24 | 139+/-24 |
| 120 | 56+/-20 | 76+/-33 | 137+/-20 | 139+/-21 |
| 240 | 62+/-22 | 68+/-32 | 136+/-26 | 134+/-28 |

V:       Clotting Factor V Activity

VIII:     c Clotting Factor VIII activity

vWag:   von Willebrand protein

R Co:   Ristocetin Cofactor

9) with reference to the results obtained with Group B, they are represented in following Tables 3b and 4b.

## Table 3a

| MIN | FIB(mg/dl) | ELT(min) | FDP(ug/ml) | D-D(ng/ml) | TT(sec) |
|---|---|---|---|---|---|
| BASAL | 308+/-99 | 140+/-56 | 3.7+/-4.7 | 31+/-12 | 19+/- 3 |
| 30 | 161+/-65 | 20+/- 9 | 132+/-165 | 48+/-26 | 42+/-27 |
| 60 | 149+/-67 | 26+/-10 | 333+/-623 | 49+/-14 | 67+/-62 |
| 120 | 157+/-68 | 51+/-20 | 338+/-632 | 60+/-26 | 104+/-80 |
| 240 | 166+/-63 | 75+/-28 | 82+/-166 | 67+/-31 | 79+/-76 |

### Table 3b

| MIN | FIB(mg/dl) | ELT(min) | FDP(ug/ml) | TT(sec) |
|---|---|---|---|---|
| BASAL | 308+/-99 | 140+/-56 | 3.7+/- 4.7 | 19+/- 3 |
| 30 | 94+/-89 | 16+/-12 | 972+/-1176 | 67+/-60 |
| 60 | 91+/-96 | 19+/-17 | 803+/- 678 | 108+/-71 |
| 120 | 90+/-93 | 26+/-23 | 605+/-537 | 118+/-67 |
| 240 | 107+/-107 | 50+/-49 | 397+/-368 | 147+/-65 |

### Table 4a

| MIN | t-PA+PAI 1 (ng/ml) | t-PA(ng/ml) | u-PA(ng/ml) | PAI-1(ng/ml) |
|---|---|---|---|---|
| BASAL | 6.5+/-2.6 | 14.2+/-6.0 | 0.58+/-0.23 | 45.1+/-15 |
| 30 | 13.7+/-5.2 | 29.7+/-7.7 | 0.42+/-0.16 | 42.0+/-23 |
| 60 | 11.4+/-6.7 | 23.3+/-3.6 | 0.39+/-0.15 | 43.7+/-20 |
| 120 | 12.3+/-4.9 | 25.2+/-7.4 | 0.37+/-0.21 | 49.4+/-14 |
| 240 | 12.1+/-7.0 | 28.7+/-9.8 | 0.39+/-0.21 | 46.5+/-10 |

### Table 4b

| MIN | t-PA+PAI 1 (ng/ml) | t-PA(ng/ml) | u-PA(ng/ml) | PAI-1(ng/ml) |
|---|---|---|---|---|
| BASAL | 6.5+/-2.6 | 14.2+/-6.0 | 0.58+/-0.23 | 45.1+/-15 |
| 30 | 12.5+/-5.7 | 23.7+/-8.8 | 0.27+/-0.18 | 42.0+/-20 |
| 60 | 8.8+/-3.8 | 17.4+/-5.2 | 0.27+/-0.13 | 41.9+/-18 |
| 120 | 7.2+/-3.4 | 15.7+/-5.8 | 0.30+/-0.16 | 44.6+/- 9 |
| 240 | 10.4+/-4.6 | 15.2+/-3.5 | 0.40+/-0.13 | 44.3+/-16 |

PAI: tissue plasminogen activator inhibitor.
u-PA: urokinase type plasminogen activator.

PAI-1 concentration remained unchanged. Tissue plasminogen activator/tissue plasminogen activator inhibitor 1 complex was above normal values before starting the therapy (6.5 +/- 2.6ng/ml; normal 1.3 +/- /0.2ng/ml). An increase of 112% was detected at 30 minutes (p 0.001) which remains between 77-91% up to the last blood sample. The free tissue plasminogen activator in plasma (functional active) increased from 7.8ng/ml at basal to 16.6ng.ml at 240 minutes (Table 5b).

Full plasminogen activation capacity which at pre-treatment is comparable to tissue plasminogen activator concentration, raised, in response to drugs infusion to a maximal of 25.2 +/- 12.3 IU/ml (p 0.001) (Table 5a) which represent an increase of 663%.

### Table 5a

| MIN | SK+DDAVP | | SK |
| | FPAC(IU/ml) | t-PA FREE (ng/ml) | FPAC(IU/ml) |
|---|---|---|---|
| BASAL | 3.3+/- 3.5 | 7.8 | 8.3+/-8 |
| 30 | 25.2+/-12.3 | 16 | 24.8+/-17 |
| 60 | 24.9+/- 6.5 | 11.9 | 16.0+/-12 |
| 120 | 22.0+/-11.6 | 12.9 | 15.4+/-15 |
| 240 | 18.8+/-11.5 | 16.6 | 14.2+/-10 |

### Table 5b

| MIN | SK+DDAVP | |
| | FPAC(IU/ml)) | t-PA FREE (ng/ml) |
|---|---|---|
| BASAL | 3.3+/- 3.5 | 7.8 |
| 30 | 25.8+/-17.2 | 11.2 |
| 60 | 16.0+/-13.8 | 8.6 |
| 120 | 9.1+/- 7.5 | 8.5 |
| 240 | 5.7+/- 7.1 | 4.8 |

SK:     "Streptokinase
FPAC:   Full Plasminogen Activation Capacity

Further results obtained on individual patients are the following:

DDAVP + STREPTOKINASE

| PATIENT A: | BASAL | 30' | 60' | 120' | 240' |
|---|---|---|---|---|---|
| ELT. | 85 | 18 | 18 | 20 | 30 |
| FIB (Clauss) | 450 | 84 | 76 | 66 | 87 |
| FIB (Gravimetric) | 460 | 160 | 200 | 210 | 200 |
| FDP | 0.5 | 256 | 128 | 128 | 128 |
| TT (normal 17') | 26 | 110 | +3' | +3' | +3' |
| FACTOR V | 80 | 42 | 45 | 55 | 55 |
| FACTOR VIII | 80 | 55 | 55 | 96 | 50 |
| BLOOD PLATELET | 200 | 200 | 160 | 180 | 200 |
| PT (Prothrombin Time) | 58 | 45 | 45 | 45 | 42 |
| FACTOR II | 60 | 56 | 56 | 56 | 56 |
| FACTOR VII-X | 80 | 62 | 62 | 58 | 58 |
| FACTOR vWillebrand Ag. | 105 | 135 | 138 | 118 | 120 |
| WHITE CELLS | 14900 | 13500 | 19000 | 19000 | 16000 |
| RED CELLS | 5560 | 5340 | 5260 | 5240 | 5320 |
| HEMOGLOBINE | 16.8 | 16.6 | 16.5 | 16.7 | 17 |
| HEMATOCRITE | 51 | 49 | 49 | 49 | 49.5 |

| PATIENT B | BASAL | 30' | 60' | 120' | 240' |
|---|---|---|---|---|---|
| ELT | 125 | 20 | 25 | 35 | 50 |
| FIB (Clauss) | 378 | 142 | 125 | 122 | 123 |
| FIB (turbidimetric) | 282 | 180 | 185 | 179 | 112 |
| FIB (gravimetric) | 380 | 240 | 230 | 230 | 200 |
| FDP | 4 | 256 | 512 | 512 | 512 |
| PT (prothrombin time) (normal 18") | 18 | 42 | 43 | 45 | 65 |
| FACTOR V | 100 | 46 | 50 | 42 | 35 |
| FACTOR VIII | | | | | |
| BLOOD PLATELET | 220 | 250 | 210 | 290 | 240 |
| PT (prothrombin time) | 66 | 53 | 50 | 39 | 42 |
| FACTOR II | 60 | 55 | 55 | 50 | 55 |
| FACTOR VII-X | 100 | 90 | 92 | 90 | 85 |
| FACTOR vWillebrand Ag | | | | | |
| WHITE CELLS | 10500 | 10200 | 12500 | 13500 | 10200 |
| RED CELLS | 5790 | 5530 | 5490 | 5270 | 5190 |
| HEMOGLOBINE | 17.1 | 16.8 | 18.9 | 17.5 | 17 |
| HEMATOCRITE | 49 | 48 | 51 | 49 | 48 |

## Claims

1. A composition for use in thrombolytic therapy, which composition comprises (A) desmopressin (also known as DDAVP) and (B) a substance selected from streptokinase, urokinase, prourokinase, acylenzymes and acylated thrombolytic agents.

2. A kit useful in the treatment of thrombolytic therapy, the kit comprising (A) desmopressin (also known as DDAVP) and (B) a substance selected from streptokinase, urokinase, prourokinase, acylenzymes and acylated thrombolytic agents, and further elements useful when supplying these ingredients.

3. Use of a combination of (A) desmospressin (also known as DDAVP) and (B) a substance selected from streptokinase, urokinase, prourokinase, acylenzymes and acylated thrombolytic agents for thrombolytic therapy.

4. Method useful in thrombolytic therapy characterised by supplying to the patient a combination of (A) desmopressin (also known as DDAVP) and (B) a substance selected from streptokinase, urokinase, prourokinase, acylenzymes and acylated thrombolytic agents.

EP 0 462 766 A1

**European Patent Office**

# PARTIAL EUROPEAN SEARCH REPORT

which under Rule 45 of the European Patent Convention shall be considered, for the purposes of subsequent proceedings, as the European search report

Application Number

EP 91 30 5428

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 98, no. 7, 14th February 1983, page 155, abstract no. 47661e, Columbus, Ohio, US; F.H.M. DERKX et al.: "Activation of plasma prorenin by plasminogen activators in vitro and increase in plasma renin after stimulation of fibrinolytic activity in vivo", & CLIN. EXP. HYPERTENS., PART A 1982, A4(11-12), 2247-58 <br> * Abstract * <br> ----- | 1-3 | A 61 K 37/54 // <br> (A 61 K 37/54 <br> A 61 K 37:34 ) |

TECHNICAL FIELDS
SEARCHED (Int. Cl.5)

A 61 K

### INCOMPLETE SEARCH

The Search Division considers that the present European patent application does not comply with the provisions of the European Patent Convention to such an extent that it is not possible to carry out a meaningful search into the state of the art of some of the claims
Claims searched completely : 1-3
Claims searched incompletely :
Claims not searched : 4
Reason for the limitation of the search:

Method for treatment of the human
or animal body by surgery or therapy
(See art. 52(4) of the European
Patent Convention)

| Place of search | Date of completion of the search | Examiner |
|---|---|---|
| THE HAGUE | 18-09-1991 | BRINKMANN C. |